Europäisches Patentamt

European Patent Office

Office européen des brevets

(11) Numéro de publication : **0 463 962 A1**

## (12) DEMANDE DE BREVET EUROPEEN

(21) Numéro de dépôt : **91401723.1**

(22) Date de dépôt : **26.06.91**

(51) Int. Cl.⁵ : **A61K 9/16**, A61K 9/50, A61K 7/00

(30) Priorité : **26.06.90 FR 9008014**

(43) Date de publication de la demande :
**02.01.92 Bulletin 92/01**

(84) Etats contractants désignés :
**AT BE CH DE DK ES FR GB GR IT LI NL SE**

(71) Demandeur : **CENTRE INTERNATIONAL DE RECHERCHES DERMATOLOGIQUES GALDERMA - CIRD GALDERMA**
**635, route des Lucioles, Sophia Antipolis**
**F-06565 Valbonne (FR)**

(72) Inventeur : **Rolland, Alain**
**126, rue des Termes**
**F-06530 - Peymeinade (FR)**

(74) Mandataire : **Stalla-Bourdillon, Bernard et al**
**CABINET NONY & CIE 29, rue Cambacérès**
**F-75008 Paris (FR)**

(54) Procédé de préparation de microsphères de corps gras chargées ou non chargées en une substance active.

(57) Nouveau procédé pour la préparation de microsphères de corps gras, non-solubles dans l'eau, chargées ou non chargées en une substance active.

Ce nouveau procédé est caractérisé par la dissolution d'au moins un corps gras choisi parmi les alcools aliphatiques ayant de 8 à 16 atomes de carbone, les acides aliphatiques ayant de 8 à 15 atomes de carbone et les esters de ceux-ci ou leurs mélanges, éventuellement avec une substance active, dans un solvant miscible dans l'eau qui peut également solubiliser ladite substance active, suivi par l'introduction, de préférence par injection à l'aide d'une seringue, de ce mélange dans un milieu aqueux sous agitation et à une température comprise entre 4 °C et le point de fusion du ou des corps gras choisi(s) et par la récupération et le séchage des micnosphères ainsi obtenues.

Utilisation des microsphères obtenues selon le procédé dans des compositions à usage thérapeutique ou cosmétique.

EP 0 463 962 A1

La présente invention a pour objet un procédé de préparation de microsphères, constituées par des corps gras, chargées ou non chargées en une substance active, et leur utilisation dans des compositions à usage thérapeutique ou cosmétique.

Un grand nombre de substances actives ne peuvent être formulées en vue d'une application quelconque du fait de leur manque de stabilité physique ou chimique, soit vis-à-vis du milieu de formulation, soit vis-à-vis des conditions de stockage en particulier vis-à-vis de l'oxygène atmosphérique ou vis-à-vis des conditions de leur préparation.

La stabilisation de substances actives dans des formulations adaptées à l'usage final et l'incorporation de substances actives chimiquement incompatibles dans une même formulation ainsi d'ailleurs que la libération progressive de la (ou des) substance(s) active(s) à partir de formulations, sont trois problèmes importants qui se posent dans ce domaine.

Jusqu'à présent, la stabilisation des substances actives a été résolue par exemple par la technique de la microencapsulation consistant à former à l'aide d'un polymère ou d'un corps gras approprié des microcapsules de la substance active.

Dans le cas des corps gras, plusieurs méthodes sont connues pour arriver à de telles microcapsules, dont certaines impliquent la formation préalable d'un mélange fondu à température élevée d'une substance lipidique et de substance active suivi par une pulvérisation dans un milieu de température réduite pour provoquer la solidification du corps gras comme revêtement autour des granules de substance active.

Pour les substances actives possédant une instabilité chimique à la chaleur, ces procédés sont contre-indiqués.

Dans le brevet britannique n° 543.309, il est décrit le revêtement de l'acide salicylique en tant que substance active par un produit lipidique obtenu par la dissolution préalable du lipide dans un solvant incapable de dissoudre la substance active suivi par l'addition de la substance active à cette solution avant d'évaporer le solvant. Ceci résulte en une masse semi-solide qui est ensuite moulée ou coupée selon la forme voulue puis séchée.

Dans le brevet américain n° 4.726.966 on a proposé l'encapsulation et la granulation de l'Ibuprofen comme agent actif dans une microsphère constituée par un polymère à savoir des résines de l'acide acrylique.

Les essais effectués par la demanderesse ont permis de démontrer que le procédé de préparation des microsphères selon le brevet américain n°4.726.966 ne conduit pas systématiquement aux microsphères attendues lorsque des corps gras sont utilisés comme produits de départ. Ceci parce que le procédé du brevet aboutit à un simple enrobage de l'Ibuprofen par la résine qui est soluble dans l'eau.

La présente invention propose un nouveau procédé préparation de microsphères de corps gras (non solubles dans l'eau) qui permet d'encapsuler des substances actives à température ambiante ce qui est avantageux pour des substances ayant une instabilité thermique.

Les différentes études qui ont été réalisées ont permis de montrer que les substances actives incorporées dans les microsphères selon l'invention étaient parfaitement stables sur de longues périodes de temps.

La présente invention a donc pour objet un procédé pour la préparation de microsphères, chargées ou non chargées en une substance active, caractérisé essentiellement par la dissolution d'au moins un corps gras ayant un point de fusion supérieur à 25 °C, choisi parmi les alcools aliphatiques ayant de 8 à 16 atomes de carbone, les acides aliphatiques ayant de 8 à 15 atomes de carbone ou les esters de ceux-ci ou leurs mélanges, avec éventuellement une substance active, dans un solvant miscible à l'eau, suivi de l'introduction de ce mélange dans un milieu aqueux, de préférence l'eau, sous agitation et à une température comprise entre 4°C et le point de fusion du ou des corps gras choisi(s) et par la récupération et le séchage des microsphères ainsi obtenues.

La présente invention a également pour objet les microsphères produites par un tel procédé.

Le diamètre moyen des microsphères s'il peut être compris dans de très larges limites, est généralement compris entre 1 et 500 $\mu$m, et de préférence entre 5 et 100 $\mu$m.

De préférence le mélange est introduit dans le milieu aqueux par injection à l'aide d'une seringue ou tout autre dispositif similaire ce qui permet un contrôle plus sensible de la grosseur des microsphères ainsi formées.

Parmi les alcools aliphatiques ayant de 8 à 15 atomes de carbone qui peuvent constituer le corps gras, ceux particulièrement préférés selon l'invention sont le tridécanol, le tétradécanol (alcool myristique), le pentadécanol et l'hexadécanol (alcool cétylique).

Parmi les acides aliphatiques ayant de 8 à 15 atomes de carbone qui peuvent constituer le corps gras, ceux particulièrement préférés selon l'invention sont l'acide undécanoïque et l'acide laurique.

Selon le procédé de l'invention, les microsphères obtenues se présentent essentiellement sous forme de particules sphériques.

Bien que l'invention ne soit pas limitée à certaines substances actives, elle présente néanmoins un intérêt tout particulier à l'égard de substances peu stables en milieu aqueux.

Parmi les substances actives qui peuvent être incorporées par formation des microsphères, on peut notamment mentionner sans que cette énumération soit limitative :

– la vitamine A, l'acide rétinoïque et ses dérivés, les vitamines E et F et leurs dérivés, l'acide 6-[3-(1-adamantyl-4) méthoxyphényl] naphtoïque-2 et ses analogues, l'acide 2-(5,6,7,8-tétrahydro-5,5,8,8- tétraméthyl-2-naphtyl)-6-benzo (b) thiophène carboxylique et ses analogues, l'acide 4-[3-(1-adamantyl)-4-méthoxybenzamido benzoïque] et ses analogues, les corticostéroïdes tels que le 17-valérate de bétaméthasone ou le 17,21-dipropionate de bétaméthasone, les anti-inflammatoires non stéroïdiens tels que l'indométhacine, le bufexamac, le N-benzylphényl- α-acétoxy- acétamide; ou d'autres dérivés du N-benzylmandélamide tels que le nicotinate de N-benzylmandélamide, le benzoate de N-benzylmandélamide, l'acétate de N-(p-chloro) benzylmandélamide, l'acétate de N-(p-méthyl) benzylmandélamide, l'acétate de N-méthyl, N-benzylmandélamide, l'O-éthoxy-carbonyl-N-benzylman-délamide ; le N-phénéthyl-diphénylacétoxyacé-tamide ; les dérivés d'acides gras polyinsaturés tels que l'acide éicosatriynoïque, les immunomodulateurs tels que les cyclosporines ou les macrolides comme les dérivés de 11,28-dioxa-4 azatricyclo 22.3.1.0.4,9 octacos-18-ene, les antihistaminiques tels que la prométbazine ou la cinnarizine, les antiprurigineux tels que l'acide palmitoyl-collagénique, les antifongiques tels que les dérivés d'éconazole ou les dérivés de pyrithione, les antipsoriasiques tels que l'anthraline et ses dérivés, les antibactériens tels que les sulfamides, les antibiotiques tels que l'érythromycine, les antiseptiques tels que les parahydroxybenzoates de propyle ou de benzyle, les antiviraux et antiherpétiques tels que la vidarabine , les anesthésiques tels que la benzocaïne, les kératolytiques tels que l'acide salicylique ou les goudrons, ou les peroxydes tels que le peroxyde de benzoyle, les agents vasodilatateurs tels que les esters de l'acide nicotinique, les agents vasculoprotecteurs ou veinotoniques tels que l'extrait de marron d'inde, les agents anticouperoses, les agents aidant à la cicatrisation, les agents anti-chutes ou favorisant la repousse des cheveux, tels que le minoxidil ou l'un de ses dérivés, etc...

L'incorporation de ces substances actives dans les microsphères permet d'en améliorer de façon particulièrement significative la stabilité physique ou chimique surtout dans le cas de substances peu stables elles-mêmes en milieu aqueux.

## PROCEDE GENERAL DE PREPARATION DES MICROSPHERES

La préparation des microsphères est réalisée par la formation préalable d'un mélange du corps gras, éventuellement d'une substance active, et d'un solvant miscible à l'eau qui peut solubiliser le corps gras ainsi que la substance active à incorporer, tel que par exemple l'éthanol, l'isopropanol ou le THF. Ceci donne une phase liquide homogène qui est ensuite introduite par simple versement mais de préférence par injection contrôlée dans la phase aqueuse, en particulier de l'eau, sous agitation, par exemple à l'aide d'un agitateur helicoïdal qui tourne à une vitesse de 100 à 1000 t/mn., et à une température d'environ 20°C. On obtient ainsi une quasi émulsion huile-dans-eau.

Le mélange du corps gras, éventuellement de la substance active, et du solvant se trouve essentiellement sous forme de gouttelettes en suspension dans la phase aqueuse, de préférence dans l'eau.

Ensuite le solvant présent dans les gouttelettes diffuse rapidement, favorisé par l'agitation, des gouttelettes vers la phase aqueuse, et les microsphères apparaissent alors selon un procédé de "cristallisation sphérique". Celles-ci peuvent être isolées par filtration puis séchage, de préférence par lyophilisation.

Le diamètre des microsphères produites est en relation inverse de la vitesse d'agitation. Il y a par ailleurs une relation directe entre le diamètre des microsphères et la longueur de la channe hydrocarbonée lorsque le corps gras est un alcool aliphatique.

Il est possible d'ajouter un tensio-actif à la phase aqueuse afin d'augmenter le diamètre des microsphères ainsi produites.

Comme tensio-actif on peut notamment utiliser ceux connus sous la dénomination de TWEEN tel que par exemple le TWEEN 80.

Le procédé est effectué de façon générale entre environ 4 et 30°C selon le point de fusion du corps gras choisi mais de préférence entre 15 et 25°C.

Dans la réalisation de l'injection à l'aide d'une seringue, il a été constaté que si la vitesse d'injection du mélange dans le milieu aqueux n'influe pas d'une manière importante sur la grosseur des microsphères, l'utilisation de la technique d'injection à l'aide d'une seringue permet cependant de maintenir un meilleur contrôle du diamètre des microsphères produites. De même, une augmentation de la concentration du solvant provoque un meilleur rendement en microsphères.

Le rapport corps gras/solvant est variable et dépend du solvant et du corps gras choisi. Cependant, il est préférable que le corps gras soit présent en une proportion de 3 à 50 % dans le solvant et de préférence supérieure à 5 %. La proportion de substance active peut être variable et dépend de celle choisie mais elle est généralement comprise entre 0,01 et 50 % et de préférence entre 0,1 et 20 % en poids du corps gras. La proportion d'eau dans la phase aqueuse est de préférence comprise entre 90 et 100 % et lorsqu'un tensio-actif est présent sa proportion est comprise entre 0,1 et 0,25 % par rapport au poids total de la phase aqueuse.

Les microsphères obtenues par le procédé selon l'invention sont destinées à la préparation de compositions pharmaceutiques notamment pour le traitement des désordres cutanés ou ophtalmologiques, ou encore pour la livraison de médicaments par voie du tractus gastroentérique, lesdites compositions contenant, dans un véhicule approprié, lesdites microsphères.

Si une application topique est désirée, on peut également incorporer dans les microsphères des substances ayant la propriété de modifier les caractéristiques physico-chimiques cutanées. On peut citer à titre d'exemple, les substances émollientes ou les substances modifiant la perméabilité de la couche cornée ou la fluidité des lipides cutanés.

Les microsphères peuvent être utilisées par voie orale pour apporter des médicaments au système gastro-intestinal humain ou animal. Les microsphères peuvent ainsi selon leur point de fusion moduler la libération des médicaments soit dans le liquide gastrique, soit en fondant de manière graduelle dans le tractus gastro-intestinale.

Dans ce dernier cas, il convient de formuler les microsphères dans des capsules entériques bien que toute autre type de formulation soit possible.

Pour des applications topiques, il est préférable d'utiliser des corps gras dont le point de fusion est inférieur à environ 55°C, de préférence inférieur à environ 40°C. En outre, si on veut livrer la substance active par voie trans-folliculaire, il convient de sélectionner un corps gras ayant une température de fusion comprise entre 40 et 50°C, tel que, par exemple, l'alcool cétylique (point de fusion = 49,2 $\pm$ 0,27 °C).

Comme indiqué précédemment, les microsphères obtenues par le procédé selon la présente invention, contiennent de 0,01 à 50 % et de préférence de 0,1 à 20 % en poids de la substance active, le reste étant essentiellement constitué par le corps gras.

Par analyse selon la méthode de KARL-FISHER et par chromatographie en phase gazeuse, on a pu en effet déterminer que l'eau et le solvant résiduel, dans les microsphères, étaient présents en une proportion inférieure à 0,1 % pour l'eau et inférieure à 0,01 % pour le solvant.

Les compositions topiques peuvent se présenter, selon le véhicule compatible avec les microsphères, sous forme d'une lotion, d'un gel aqueux hydroalcoolique ou hydroglycolique, d'une formulation à base de silicone, d'une poudre (la charge étant de l'oxyde de zinc et/ou du talc) ou d'une pommade. A ce sujet, il est notamment possible d'utiliser un véhicule topique dans lequel la substance active elle-même n'est guère soluble.

Parmi les agents épaississants utilisables pour la formation des gels, on peut notamment citer les polymères carboxyvinyliques tels que ceux vendus sous la dénomination de "CARBOPOL" par la Société GOODRICH BF, notamment le "CARBOPOL 940" ou le "CARBOPOL 930" ou les dérivés de cellulose tels que l'hydroxypropylcellulose vendu sous la dénomination de "KLUCEL HF" par la Société HERCULES.

Les microsphères sont généralement présentes dans les compositions en une proportion pouvant être comprise entre 1 à 50 % en poids par rapport au poids total de la composition.

Comme ceci a été indiqué précédemment, ces compositions peuvent contenir des mélanges de microsphères chargées séparément en substances actives incompatibles ou encore contenir dans la phase dispersante (aqueuse) une substance active à l'état dissous ou dispersé.

Par application topique de ces compositions, par un léger massage sur la peau ou les muqueuses, les microsphères chargées en substance active fondent progressivement donnant un système favorable à une action progressive de la substance active sur les tissus ciblés sans agression de celle-ci.

On va maintenant donner à titre d'illustration et sans aucun caractère limitatif plusieurs exemples de préparation des microsphères selon l'invention, chargées ou non chargées en une substance active, ainsi que des exemples de compositions pharmaceutiques.

## EXEMPLE 1

Microsphères chargées en
N-(hydroxy-2-éthyloxyéthyl) eicosatriynamide.

(a) Dans 3ml d'éthanol sont dissous 0,5 g de tridécanol (point de fusion = 31,1 $\pm$ 0,1°C) et 32mg de N-(hydroxy-2-éthyloxyéthyl) eicosatriynamide (6 % en poids par rapport au poids total du mélange corps gras et substance active).

Le mélange a alors été injecté dans 100 ml d'eau déminéralisée sous agitation.

Les microsphères ainsi formées ont alors été lavées à l'eau déminéralisée puis séchées.

Par chromatographie liquide de haute performance (HPLC) on a pu constater que 83 % de la substance active était chargée dans les microsphères.

(b) On a suivi le même protocole que pour l'exemple 1(a) sauf mue l'on a utilisé du tétradécanol (point de fusion = 38,1 $\pm$ 0,2°C) au lieu du tridécanol. Ceci a conduit à l'incorporation de 85 % de la substance active dans les microsphères.

## EXEMPLE 2

Microsphères chargées en acide 6- 3-
(1-adamantyl)-4 méthoxyphényl -2-phtanoïque

Dans 3ml d'éthanol sont dissous 0,5g d'alcool gras (tri ou tétradécanol et 4mg d'acide 6- 3-(1-adamantyl)-4-méthoxyhényl - 2-naphtoïque (0,8 % en poids) et dans les mêmes conditions opératoires que pour l'exemple 1(a) et (b) on arrive à l'aide de tridécanol et de tétradécanol à un taux d'incorporation de la substance active de 94 % et 100 % respectivement déterminé par HPLC.

## EXEMPLE 3

Microsphères chargées en acide rétinoïque

(i) Dans un premier essai 0,5g de tridécanol et 20mg d'acide rétinoïque (3,8 % en poids) ont été dissous dans 5 ml d'éthanol absolu. Dans un deuxième essai 0,5g de tétradécanol ont été utilisés à la place de tridécanol. Chaque mélange a alors été injecté dans 100ml d'eau distillée pour conduire à des microsphères qui ont été lavées deux fois à l'eau déminéralisée puis séchées. Ensuite la teneur d'incorporation de l'acide rétinoïque a été mesurée par HPLC.

Pour le tridécanol la teneur d'incorporation était de 74 % et pour le tétradécanol de 61 %.

(ii) Dans une préparation similaire, 2 % en poids d'acide rétinoïque a été chargé dans des microsphères de tri- et de tétradécanol ayant respectivement un taux d'incorporation de 66,5 % et 61 %.

(iii) Dans une variation du procédé, 51 mg d'acide rétinoïque

et 2,5 g de tétradécanol sont dissous dans 25 ml d'éthanol absolu contenant également 0,04 % de butylhydroxytoluène (BHT). Le mélange éthanolique a alors été lentement injecté au moyen d'une seringue (du type aiguille : 23 G x 1") dans 500 ml d'une solution aqueuse contenant 0,01 % de 3-tert-butyl-4-hydroxyanisole (BHA) à 20 °C et sous agitation à une vitesse de 300 t/mn. La préparation a été effectuée sous lumière jaune.

Des microsphères ayant un domaine de diamètre compris entre 5 et 70 μm ont été obtenues.

Ensuite les microsphères ont été lavées 3 fois avec une solution aqueuse de BHA à 0,01 % puis séchées par lyophilisation. Le dosage par HPLC de l'acide rétinoïque confirme son incorporation dans les microsphères (1,9 % en poids) et montre qu'aucune dégradation ne s'est produite.

(iv) Une formulation topique dosée à 0,1 en acide rétinoïque a été préparée par incorporation, sous lumière jaune, de microsphères chargées à 1,9 % en poids d'acide rétinoïque, dans un gel aqueux à 0,25 % de Carbopol 940, contenant 0,01 % de BHA.

Après 6 mois à +4°C, la bonne stabilité des microsphères dans le gel aqueux a été vérifiée par microscopie optique. Un dosage HPLC a également mis en évidence la stabilité chimique de l'acide rétinoïque encapsulée (aucune dégradation).

## EXEMPLE 4

Microsphères chargées en anthraline

0,5 g de tétradécanol et 10g d'anthraline (2 % en poids), sont dissous dans 5 ml d'éthanol. La solution éthanolique a alors été injectée dans 100 ml d'eau déminéralisée à une vitesse de révolution de 750 t/mn.

Les microsphères obtenues ont ensuite été lavées deux fois à l'eau déminéralisée puis mise en suspension dans l'eau.

Afin de démontrer la stabilité dans le temps de ces microsphères celles-ci ont été maintenues un mois à 4°C dans ladite suspension aqueuse puis filtrées et analysées par HPLC pour quantifier les produits de dégradation de l'anthraline.

Comme témoin de l'anthraline a été dissoute dans de l'éthanol et la solution injectée dans de l'eau et le mélange a été également maintenu dans les mêmes conditions de temps et de température.

L'anthraline pure (non sous forme de microsphères) dans l'eau s'est dégradée en quinone correspondante caractérisée par une couleur brune intense. Par contre, les microsphères de tétradécanol chargées en anthraline sont restées jaune pendant la même période de temps ce qui indique que la dégradation était beaucoup moins importante.

(ii) Dans un mode de préparation similaire, 2,5 g de tétradécanol et 25,5 mg d'anthraline (1 % en poids) sont dissous sous lumière jaune dans 25 ml d'éthanol absolu contenant également 0,04 % de BHT.

La solution éthanolique a été injectée manuellement dans une solution aqueuse contenant 0,01 % de BHA à 20 °C et sous agitation à 300 t/mn.

Après 30 min d'agitation, les microsphères ainsi produites ont été lavées trois fois avec la même solution aqueuse à 0,01 % de BHA et une fois avec de l'eau déminéralisée.

Les microsphères chargées en anthraline ont ensuite été séchées par lyophilisation. La teneur en anthraline dans les microsphères à été déterminée par HPLC comme étant de 0,84 % et aucun produit de dégradation n'a été détecté.

## EXEMPLE 5

Microsphères chargées en N-benzylphényl acétoxyacétamide.

En suivant le même mode opératoire que ci-dessus, 15 % en poids total de N-benzylphényl acétoxyacétamide a été chargé dans des microsphères de tétradécanol.

EXEMPLE 6

Microsphères chargées en minoxidil.

En suivant le même mode opératoire que ci-dessus, 5 % en poids total de minoxidil a été chargé dans des microsphères de tétradécanol.

EXEMPLE 7

Microsphères chargées en peroxyde de benzoyle.

0,5 g de tétradécanol et 0,55 g en poids de peroxyde de benzoyle (10 % en poids) sont dissous dans 5 ml d'éthanol. Ce mélange éthanolique a alors été injecté dans 100 ml d'eau déminéralisée sous agitation.

Après séparation, les microsphères obtenues sont observées au microscope et l'or constate qu'il n'y a pas de cristaux de peroxyde de benzoyle à l'extérieur des microsphères. La totalité du peroxyde de benzoyle est donc chargé dans les microsphères.

Dans les exemples 1 à 7 précédents, où soit le tridécanol soit le tétradécanol a été pris comme corps gras, une réduction de la vitesse d'agitation de 750 à 250 t/mn provoque une croissance moyenne des microsphères de 39 à 77 μm pour le tridécanol et de 55 à 105 μm pour le tétradécanol.

Les diamètres ont été mesurés à l'aide de l'appareil Malvern MASTERSIZER.

EXEMPLE 8

Microsphères non-chargées de pentadécanol

(a) 0,5 g de pentadécanol (point de fusion = 44,0 ± 0,12 °C) est dissous respectivement (i) dans 10 ml et (ii) dans 15 ml d'éthanol et les mélanges obtenus ont été injectés dans 100 ml d'eau déminéralisée à 20 °C et sous une agitation de 500 t/mn.

Les microsphères obtenues à l'aide de 10 ml d'éthanol avaient un diamètre de 1-20 μm avec très peu de débris.

Les microsphères obtenues à l'aide de 15 ml d'éthanol avaient un diamètre de 1 à 7 μm et étaient très homogènes.

Il résulte de cette comparaison qu'une augmentation de la concentration en éthanol a pour conséquence une diminution des débris ainsi qu'une réduction de la taille des microsphères.

(b) Le même mode opératoire que dans l'exemple 8 (a) avec 15 ml d'éthanol a été suivi sauf que l'on a ajouté à la phase aqueuse un tensio-actif non ionique (TWEEN 80) ce qui a augmenté les domaines de diamètre des microsphères de 5 à 45 μm pour 0,25 % de tensio-actif et de 5 à 100 μm pour 0,10 % de tensio-actif.

Le même effet a été observé avec le tri- et le tétradécanol.

L'emploi d'un tensio-actif non ionique peut donc être utile pour augmenter les domaines de diamètre des microsphères.

EXEMPLE 9

Microsphères chargées en Ibuprofen

On a suivi le même type de protocole expérimental que ci-dessus. Des microsphères contenant de 1 %, 2 % et 5 % d'Ibuprofen et respectivement 99 %, 98 % et 95 % de tétradécanol ont été obtenues. L'emploi de 85 % d'Ibuprofen avec 15 % de tétradécanol a conduit à une sédimentation de cristaux sans qu'il y ait formation de microsphères.

EXEMPLE 10

Microsphères non-chargées d'acide undécanoïque

0,25 g d'acide undécanoïque (point de fusion = 26-30°C) a été solubilisé dans 2,5 ml d'alcool éthylique absolu. Cette solution a été injectée lentement, sous agitation, dans 100 ml d'eau distillée.

Après quelques minutes d'agitation, des sphères d'un diamètre de 15 à 60 μm ont été obtenues.

EXEMPLE 11

Microsphères non-chargées d'acide laurique

0,5 g d'acide laurique (point de fusion = 42-45°C) a été solubilisé dans 3 ml d'alcool éthylique absolu. Cette solution a été injectée lentement dans 100 ml d'eau distillée sous agitation.

Des microsphères, d'un diamètre de 5 à 100 μm (moyenne 40-50 μm) ont été obtenues.

**Revendications**

1. Procédé pour la préparation de microsphères de corps gras, chargées ou non chargées en une substance active, caractérisé par la dissolution d'au moins un corps gras choisi parmi les alcools aliphatiques ayant de 8 à 16 atomes de carbone, les acides aliphatiques ayant de 8 à 15 atomes de carbone et les esters de ceux-ci ou leurs mélanges, avec éventuellement une substance active, dans un solvant miscible à l'eau, suivi de l'introduction de ce mélange dans un milieu aqueux sous agitation et à une température comprise entre 4 °C et le point de fusion du ou des corps gras choisi(s) et par la récupération et le séchage des microsphères ainsi obtenues.

2. Procédé selon la revendication 1, caractérisé par le fait que le corps gras est présent, dans ledit mélange, à une concentration de 3 à 50 % et de préférence d'au moins 5 %.

3. Procédé selon la revendication 1 ou 2, caractérisé par le fait que ladite substance active est présente dans les microsphères en une proportion inférieure à 50 % et de préférence inférieure à 20 % en poids.

4. Procédé selon l'une quelconque des revendications 1 à 3, caractérisé par le fait que le diamètre des microsphères est de l'ordre de 1 à 500 μm et de préférence de 5 à 100 μm.

5. Procédé selon l'une quelconque des revendications 1 à 4, caractérisé par le fait que l'on introduit ledit mélange dans le milieu aqueux par injection à l'aide d'une seringue.

6. Procédé selon l'une quelconque des revendications 1 à 5, caractérisé par le fait que les alcools aliphatiques ayant de 8 à 16 atomes de carbone sont choisis parmi le tridécanol, le tétradécanol, le pentadécanol et l'héxadécanol.

7. Procédé selon l'une quelconque des revendications 1 à 5, caractérisé par le fait que les acides aliphatiques ayant de 8 à 15 atomes de carbone sont choisis parmi l'acide undécanoïque et l'acide laurique.

8. Procédé selon l'une quelconque des revendications 1 à 7, caractérisé par le fait que le solvant est choisi parmi l'éthanol, l'isopropanol et le THF.

9. Procédé selon l'une quelconque des revendications 1 à 8, caractérisé par le fait que le milieu aqueux contient également un agent tensio-actif.

10. Procédé selon la revendication 9 caractérisé par le fait que le tensio-actif est présent en une proportion comprise entre 0,1 et 0,25 % en poids par rapport au poids total de la phase aqueuse.

11. Microsphères telles qu'obtenues par le procédé selon les revendications 1 à 10.

12. Utilisation des microsphères telles qu'obtenues par le procédé selon les revendications 1 à 10 dans des compositions à usage thérapeutique ou cosmétique.

**Office européen
des brevets**

# RAPPORT DE RECHERCHE EUROPEENNE

Numero de la demande

EP 91 40 1723

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | US-A-4 726 966 (Y. KAWASHIMA et al.) <br> * Le document en entier * <br> --- | 1-5,8-12 | A 61 K 9/16 <br> A 61 K 9/50 <br> A 61 K 7/00 |
| Y | US-A-4 492 720 (B. MOSIER) <br> * Colonne 2, lignes 8-33; colonne 2, ligne 66 - colonne 4, ligne 50; colonnes 5,6, exemples 1,2; revendications 1,2 * <br> --- | 1-5,8-12 | |
| A | FICHE DOCUMENTAIRE DE LA FIRME ROHM PHARMA GmbH, Darmstadt, DE; "Eudragit S" <br> * Introduction: "Auflösungsvorschrift" * <br> --- | 1 | |
| A | LABO-PHARMA - PROBL. TECH., vol. 32, no. 339, février 1984, pages 105-109, Paris, FR; P. TILLEUL et al.: "Mise au point et étude de microsphères d'alcool cétylique pour embolisation contenant un antimitotique: la Lomustine" <br> * Page 106, paragraphe 2: "Principe de fabrication" * <br> ----- | 6 | **DOMAINES TECHNIQUES RECHERCHES (Int. Cl.5)** <br><br> A 61 K <br> B 01 J |

Le présent rapport a été établi pour toutes les revendications

| Lieu de la recherche | Date d'achèvement de la recherche | Examinateur |
|---|---|---|
| LA HAYE | 23-09-1991 | BOULOIS D.J-M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P0402)